# EUROPÄISCHE PATENTSCHRIFT

(11) **EP 3 483 151 B1**
(45) Veröffentlichungstag und Bekanntmachung des Hinweises auf die Patenterteilung: **25.06.2025**
(21) Anmeldenummer: 18206125.9
(22) Anmeldetag: 04.03.2016
(51) Int. Cl.: C07D 309/12

(54) **TETRAHYDROPYRANYLESTER ALS RIECHSTOFFE**
TETRAHYDROPYRANYL ESTERS AS ODORANTS
ESTERS DE TÉTRAHYDROPYRANYL COMME MATIÈRES ODORANTES

(30) Priorität: 05.03.2015 EP 15157835
(43) Veröffentlichungstag der Anmeldung: 15.05.2019
(62) Teilanmeldung aus: 16707808.8
(73) Patentinhaber: BASF SE, 67056 Ludwigshafen am Rhein (DE)
(72) Erfinder: RUEDENAUER, Stefan, DE- 69469 Weinheim (DE); STORK, Timon, DE- 68642 Buerstadt Bobstadt (DE); PELZER, Ralf, DE - 37699 Fuerstenberg (DE); HICKMANN, Volker, DE- 67063 Ludwigshafen (DE); KLOS, Margarethe, DE - 67240 Bobenheim Roxheim (DE)
(74) Vertreter: BASF IP Association

(56) Entgegenhaltungen:
- EP-A2- 0 383 446
- EP-A2- 0 383 446
- US-A1- 2009 263 336
- AGNESE ABATE ET AL: "Preparation of the Enantiomerically Enriched Isomers of the Odorous Cyclic EthersClarycet? ,Florol? , andRhubafuran? by Enzymatic Catalysis", HELVETICA CHIMICA ACTA, vol. 87, no. 4, 1 April 2004 (2004-04-01), pages 765 - 780, XP055564013, ISSN: 0018-019X, DOI: 10.1002/hlca.200490075

## Beschreibung

### HINTERGRUND DER ERFINDUNG

Die vorliegende Erfindung betrifft bestimmte Tetrahydropyranylester der Formel (1.1) gemäß unterer Definition und ihre Verwendung als Riechstoffe.

### STAND DER TECHNIK

Zur Herstellung von Konsum- und Verbrauchsgütern mit bestimmten organoleptischen Eigenschaften, d. h. von Produkten, die über vorteilhafte geruchliche (olfaktorische) oder geschmackliche (gustatorische) Eigenschaften verfügen, steht eine große Vielzahl an Aromachemikalien (Riech- und Geschmacksstoffen) für die äußerst diversen Einsatzbereiche dieser Stoffe zur Verfügung.

Es ist bekannt, diverse substituierte Tetrahydropyranverbindungen als Aromachemikalien zu verwenden. So sind beispielsweise 2,4,4-substituierte Tetrahydropyranylester der allgemeinen Formel (A) wertvolle Aromachemikalien:

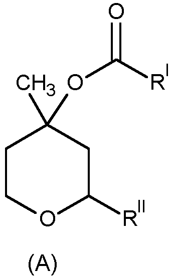

Die EP 0383446 A2 beschreibt die Synthese sowie die olfaktorischen Eigenschaften einer Vielzahl verschiedener 2,4,4-trisubstituierter Tetrahydropyranylester (A), worin R^{I} für Methyl oder Ethyl steht und R^{II} für geradkettiges oder verzweigtes C₂-C₄-Alkyl oder C₂-C₄-Alkenyl steht. Dazu wird zunächst 3-Methylbut-3-en-1-ol mit einem Aldehyd der Formel R^{II}-CHO in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (B) enthält:

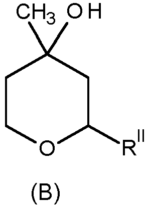

Die Zwischenstufe (B) wird anschließend einer Acylierung durch Umsetzung mit einem Carbonsäureanhydrid unter sauren Bedingungen unterzogen.

4-Hydroxy-tetrahydropyranverbindungen und speziell 2-substituierte 4-Hydroxy-4-methyl-tetrahydropyrane sind ebenfalls wertvolle Verbindungen für den Einsatz als Aromachemikalien, und dem Fachmann sind diverse Verfahren zu ihrer Herstellung bekannt, z. B. aus der EP 1 493 737 A1, WO 2011/147919, WO 2010/133473, WO 2011/154330 und WO 2014/060345.

### ZUSAMMENFASSUNG DER ERFINDUNG

Gegenstand der Erfindung sind Tetrahydropyranylester der allgemeinen Formel (1.1) 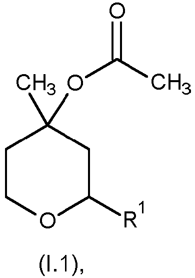 worin
R¹ für Phenyl oder mit C₁-C₆-Alkyl oder C₁-C₆-Alkoxy substituiertes Phenyl steht.

Gegenstand der Erfindung ist außerdem die Verwendung dieser Verbindungen (1.1) als Riechstoffe.

Die Verbindungen (I.1) sind dadurch erhältlich, dass man wenigstens ein 2-substituiertes 4-Hydroxy-4-methyl-tetrahydropyran der allgemeinen Formel (II.1) bereitstellt 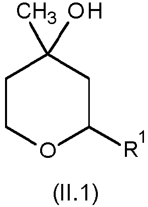 wobei R¹ die zuvor angegebenen Bedeutungen hat, und die Verbindung der allgemeinen Formel (II.1) einer Umsetzung mit dem Keten (III.1)

CH₂=C=O (III.1)

unterzieht.

### BESCHREIBUNG DER ERFINDUNG

Sofern im Folgenden nicht genauer angegeben, bezeichnen die Begriffe
"Tetrahydropyranylester",
"4-Hydroxy-tetrahydropyranverbindung",
"2-substituiertes 4-Hydroxy-4-methyl-tetrahydropyran",
"2-substituiertes 4-Methyl-tetrahydropyranyl-4-acetat"

im Rahmen der Erfindung cis/trans-Gemische jedweder Zusammensetzung sowie die reinen Konformations-Isomere. Die zuvor genannten Begriffe bezeichnen weiterhin alle Enantiomere in Reinform sowie racemische und optisch aktive Gemische der Enantiomeren dieser Verbindungen.

Sofern im Folgenden von cis- und trans-Diastereomeren der Verbindungen (I.1) die Rede ist, wird jeweils nur eine der enantiomeren Formen abgebildet. Lediglich zur Veranschaulichung werden im Folgenden beispielhaft die Isomeren des 2-Isobutyl-4-methyl-tetrahydropyran-4-yl-acetats (I.1a) wiedergegeben (anstelle der Isobutylgruppe steht in Verbindungen (I.1) allerdings ein Phenylring oder ein mit C₁-C₆-Alkyl oder C₁-C₆-Alkoxy substituierter Phenylring):

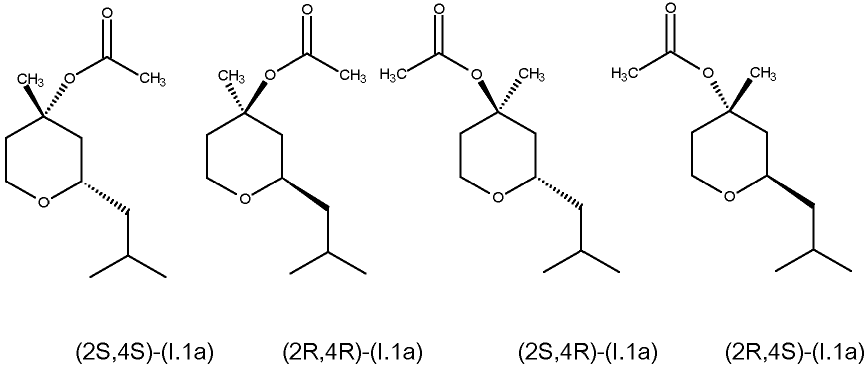

Im Rahmen der vorliegenden Erfindung steht C₁-C₆-Alkyl insbesondere für Methyl, Ethyl, n-Propyl, Isopropyl, n-Butyl, Isobutyl (2-Methylpropyl), sec.-Butyl (1-Methylpropyl), tert.-Butyl (1,1-Dimethylethyl), n-Pentyl oder n-Hexyl. Speziell steht C₁-C₆-Alkyl für Methyl, Ethyl, n-Propyl, Isopropyl, oder Isobutyl.

Im Rahmen der vorliegenden Erfindung steht C₁-C₆-Alkoxy insbesondere für Methoxy, Ethoxy, n-Propyloxy, Isopropyloxy, n-Butyloxy, Isobutyloxy, sec.-Butyloxy, tert.-Butyloxy, n-Pentyloxy oder n-Hexyloxy. Speziell steht C₁-C₆-Alkoxy für Methoxy, Ethoxy, n-Propyloxy, Isopropyloxy, oder Isobutyloxy.

Substituiertes Phenyl kann einen oder mehrere (z. B. 1, 2, 3, 4 oder 5) Substituenten aufweisen. Diese sind ausgewählt unter C₁-C₆-Alkyl und C₁-C₆-Alkoxy. Beispiele für substituierte Phenylreste sind 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl und 2,4,6-Tri-tert.-butylphenyl.

In einer bevorzugten Ausführung steht R¹ für Phenyl.

In einer weiteren bevorzugten Ausführungsform sind die Verbindungen (I.1) ausgewählt unter Verbindungen der Formeln (I-A.1), (I-A.6), (I-A.7), (I-A.8) und (I-A.9):

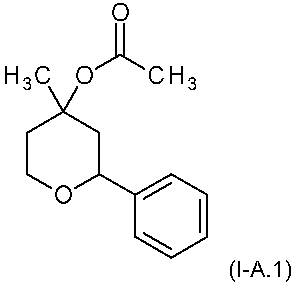

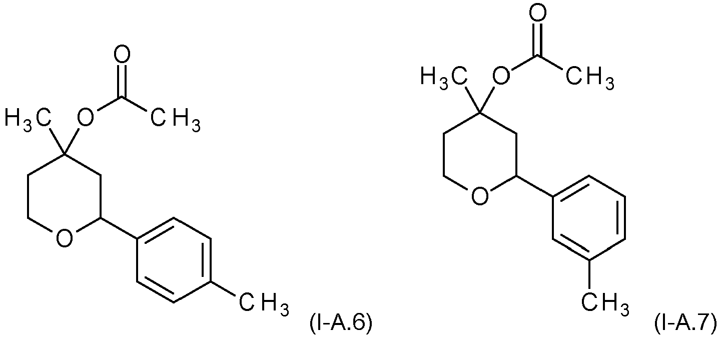

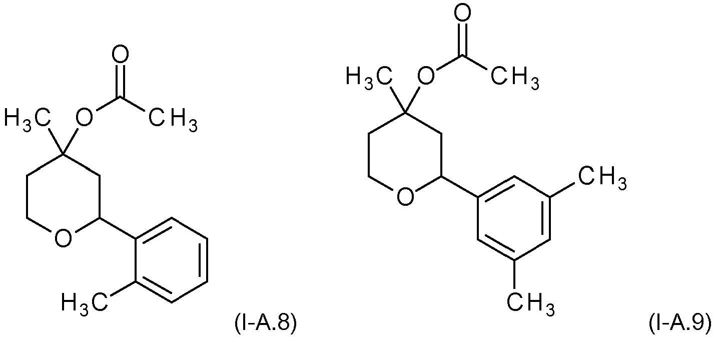

Für den Einsatz im oben beschriebenen Verfahren (das nicht Teil der vorliegenden Erfindung ist) geeignete 4-Hydroxy-tetrahydropyranverbindungen der allgemeinen Formel (II.1) und Verfahren zu deren Herstellung sind dem Fachmann prinzipiell bekannt.

Hierbei wird ein 2-substituiertes 4-Hydroxy-4-methyl-tetrahydropyran der allgemeinen Formel (II.1) eingesetzt 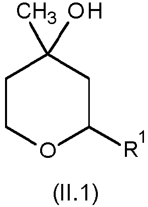 wobei R¹ die zuvor angegebene Bedeutung hat.

Vorzugsweise wird zur Bereitstellung des 2-substituierten 4-Hydroxy-4-methyl-tetrahydropyrans der allgemeinen Formel (II.1)
a) 3-Methylbut-3-en-1-ol der Formel (IV) 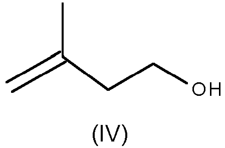 mit einem Aldehyd der Formel (V)

   R¹-CHO (V)

   worin
   R¹ die zuvor angegebene Bedeutungen hat,
   in Gegenwart eines sauren Katalysators umgesetzt, wobei ein Reaktionsgemisch erhalten wird, das wenigstens ein 2-substituiertes 4-Hydroxy-4-methyl-tetrahydropyran der allgemeinen Formel (II.1) enthält, worin R¹ die zuvor angegebene Bedeutung hat,
b) gegebenenfalls das Reaktionsgemisch aus Schritt a) einer Auftrennung unter Erhalt wenigstens einer an den 2-substituierten 4-Hydroxy-4-methyl-tetrahydropyranen der allgemeinen Formel (II.1) angereicherten Fraktion unterzogen wird.

Derartige Verfahren sind z. B. in der EP 1 493 737 A1, WO 2011/147919, WO 2010/133473, WO 2011/154330 und WO 2014/060345 beschrieben.

In einer speziellen Ausführungsform wird das Reaktionsgemisch aus Schritt a) einer Auftrennung unter Erhalt wenigstens einer an den 2-substituierten 4-Hydroxy-4-methyl-tetrahydropyranen der allgemeinen Formel (1.1) angereicherten Fraktion und einer an den 2-substituierten 4-Hydroxy-4-methyl-tetrahydropyranen der allgemeinen Formel (1.1) abgereicherten Fraktion unterzogen (= Schritt b)).

Einer der Ausgangsstoffe für Schritt a) des Verfahrens ist 3-Methylbut-3-en-1-ol (Isoprenol) der Formel (IV),

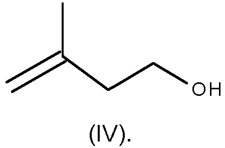

Isoprenol ist nach bekannten Verfahren aus Isobuten und Formaldehyd in jedem Maßstab gut zugänglich und kommerziell verfügbar. An die Reinheit, Qualität oder Herstellverfahren des einzusetzenden Isoprenols sind keine besonderen Anforderungen zu stellen. Es kann in handelsüblicher Qualität und Reinheit in Schritt a) des Verfahrens eingesetzt werden. Bevorzugt setzt man Isoprenol ein, das eine Reinheit von 90 Gew.-% oder darüber hat, besonders bevorzugt solches mit einer Reinheit von 95 bis 100 Gew.-% und ganz besonders bevorzugt solches mit einer Reinheit von 97 bis 99,9 Gew.-% oder noch mehr bevorzugt 98 bis 99,8 Gew.-%.

Ein weiterer Ausgangsstoff für Schritt a) des Verfahrens ist ein Aldehyd der Formel (V) R¹-CHO, wobei R¹ in der Formel (V) die zuvor angegebene Bedeutung hat.

Ein bevorzugt einzusetzender Aldehyd der Formel (V) ist Benzaldehyd.

Bevorzugt werden in Schritt a) das 3-Methylbut-3-en-ol (IV) und der Aldehyd (V) in einem molaren Verhältnis von etwa 1 zu 2 bis 2 zu 1, besonders bevorzugt von 0,7 zu 1 bis 2 zu 1, insbesondere von 1 zu 1 bis 2 zu 1, eingesetzt. In einer speziellen Ausführung werden in Schritt a) das 3-Methylbut-3-en-ol (IV) und der Aldehyd (V) in einem molaren Verhältnis von 1 zu 1 bis 1,5 zu 1 eingesetzt.

Bevorzugt erfolgt die Umsetzung in Schritt a) in Gegenwart eines sauren Katalysators. Prinzipiell kann für die Umsetzung in Schritt a) jeder saure Katalysator verwendet werden, d. h. jede Substanz, die Brönstedt- oder Lewis-Acidität aufweist. Beispiele für geeignete Katalysatoren sind Protonensäuren, wie Salzsäure, Schwefelsäure, Phosphorsäure, Methansulfonsäure und p-Toluolsulfonsäure, saure molekulare Elementverbindungen, wie Aluminiumchlorid, Bortrifluorid, Zinkchlorid, Zinntetrachlorid und Titantetrachlorid; oxidische saure Festkörper wie Zeolithe, Silikate, Aluminate, Alumosilikate, Tone und stark saure Ionentauscher.

Unter dem Begriff stark saurer Kationenaustauscher wird dabei ein Kationenaustauscher in der H⁺-Form verstanden, der stark saure Gruppen aufweist. Bei den stark sauren Gruppen handelt es sich in der Regel um Sulfonsäuregruppen. Die sauren Gruppen sind in der Regel angebunden an eine Polymermatrix, die z. B. gelförmig bzw. makroporös sein kann. Eine bevorzugte Ausführungsform des Verfahrens ist dementsprechend dadurch gekennzeichnet, dass man einen stark sauren, Sulfonsäuregruppen aufweisenden Kationenaustauscher einsetzt. Geeignete stark saure Kationenaustauscher sind in der WO 2010/133473 und WO 2011/154330 beschrieben.

Die Umsetzung in Schritt a) kann wahlweise auch zusätzlich in Gegenwart eines unter den Reaktionsbedingungen inerten externen organischen Lösungsmittels durchgeführt werden. Geeignete Lösungsmittel sind beispielsweise tert-Butylmethylether, Cyclohexan, Dekalin, Hexan, Heptan, Ligroin, Petrolether, Toluol oder Xylol. Die genannten Lösungsmittel können alleine oder in Form von Gemischen untereinander eingesetzt werden. Bevorzugt führt man die Umsetzung in Schritt a) ohne Zusatz eines externen organischen Lösungsmittels durch.

Bevorzugt wird in Schritt b) das Reaktionsgemisch aus Schritt a) einer destillativen Auftrennung unter Erhalt wenigstens einer an den 2-substituierten 4-Hydroxy-4-methyl-tetrahydropyranen der allgemeinen Formel (II.1) angereicherten Fraktion und einer an den 2-substituierten 4-Hydroxy-4-methyl-tetrahydropyranen der allgemeinen Formel (II.1) abgereicherten Fraktion unterzogen. Geeignete Vorrichtungen zur destillativen Auftrennung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer (agitated thin-film evaporator), etc. und Kombinationen davon. Die Destillationskolonnen können trennwirksame Einbauten aufweisen, die vorzugsweise ausgewählt sind unter Trennböden, geordnete Packungen, z. B. Blech- oder Gewebepackungen, wie Sulzer Mellapak^{®}, Sulzer BX, Montz B1 oder Montz A3 oder Kühni Rombopak, oder regellose Schüttungen von Füllkörpern, wie z. B. Dixon-Ringen, Raschig-Ringen, High-Flow-Ringen oder Raschig-Super-Ringen. Ein bevorzugtes Verfahren zur Herstellung und Isolierung von 2-sustituierten 4-Hydroxy-4-methyl-tetrahydropyranolen durch Umsetzung von 3-Methylbut-3-en-1-ol (Isoprenol) mit den entsprechenden Aldehyden in Gegenwart eines stark sauren Kationenaustauschers und anschließender Isolierung bzw. destillativer Abtrennung in einer Trennwandkolonne oder in einer Zusammenschaltung von zwei Destillationskolonnen in Form einer thermischen Kopplung ist in der WO 2011/154330 beschrieben.

Im Verfahren, mit welchem Verbindungen (1.1) erhältlich sind, wird wenigstens eine 4-Hydroxy-tetrahydropyranverbindung der allgemeinen Formel (II.1) mit einem Keten der Formel (III.1) (CH₂=C=O (Ethenon)) umgesetzt.

Bevorzugt erzeugt man das Keten (III.1) durch Hochtemperatur-Pyrolyse von Aceton oder Essigsäure bei Temperaturen, die in der Regel höher als 650 °C sind. Bevorzugt liegt die Temperatur zur Erzeugung des Ketens (III.1) in einem Bereich von 650 bis 1000 °C, besonders bevorzugt von 700 bis 900 °C.

In einer speziellen Ausführungsform wird das Keten (III.1) unter vermindertem Druck hergestellt. Bevorzugt liegt der Druck in einem Bereich von etwa 100 bis 900 mbar, besonders bevorzugt von 300 bis 500 mbar, insbesondere von 350 bis 450 mbar. In einer alternativen Ausführungsform wird das Keten (III.1) unter Umgebungsdruck ("drucklos") hergestellt. Bevorzugt liegt der Druck dann in einem Bereich von etwa 950 bis 1050 mbar.

Da das Keten (III.1) eine äußerst reaktive Verbindung ist, die stark zur Dimerisierung unter Bildung von Diketenen neigt, verwendet man im Verfahren Keten (III.1), welches unmittelbar vor der Umsetzung im Verfahren hergestellt worden ist, z. B. durch thermische Spaltung von Aceton, Essigsäure oder Essigsäureanhydrid oder durch Dehydrochlorierung von Acetylchlorid mit Basen, wie Triethylamin.

In einer ersten Variante des Verfahrens wird das Keten (III.1) unter der Flüssigkeitsoberfläche in das Reaktionsgemisch eingeführt, so dass es das Reaktionsgemisch durchperlt. Vorteilhafterweise wird das Keten unter intensivem Rühren in das Reaktionsgemisch geführt, so dass im Wesentlichen kein Keten in größeren Mengen in die Gasphase übertritt. Der Druck des Ketens (III.1) muss ausreichend hoch sein, um den hydrostatischen Druck des Reaktionsgemisches oberhalb der Keten-Einspeisung zu überwinden, gegebenenfalls unterstützt durch einen Inertgasstrom, z. B. Stickstoff.

Das Keten (III.1) kann über beliebige geeignete Vorrichtungen eingespeist werden. Wichtig sind dabei eine gute Verteilung und eine schnelle Durchmischung. Es eignen sich z. B. Begasungslanzen, die fest installiert sein können, oder vorzugsweise Düsen. Die Düsen können am oder in der Nähe des Reaktorbodens vorgesehen sein. Die Düsen können hierzu als Öffnungen einer den Reaktor umgebenden Hohlkammer ausgebildet sein. Bevorzugt werden jedoch Tauchdüsen mit geeigneten Zuleitungen eingesetzt. Mehrere Düsen können z. B. in Form eines Kranzes angeordnet sein. Die Düsen können nach oben oder nach unten weisen. Die Düsen weisen vorzugsweise schräg nach unten.

In einer zweiten Variante des Verfahrens wird das Keten (III.1) unter vermindertem Druck hergestellt und unter vermindertem Druck mit wenigstens einer 4-Hydroxy-tetrahydropyranverbindung der allgemeinen Formel (II.1) umgesetzt. Bevorzugt liegt der Druck bei der Herstellung und der Umsetzung des Ketens (III.1) in einem Bereich von etwa 100 bis 900 mbar, besonders bevorzugt von 300 bis 500 mbar, insbesondere von 350 bis 450 mbar.

Verfahren und Vorrichtungen zur Herstellung von Ethenon sind z. B. in Organic Syntheses, Coll. Vol. 1, S. 330 (1941) und Vol. 4, S. 39 (1925) sowie in der Chemiker Zeitung 97, Nr. 2, Seiten 67 bis 73 (1979) beschrieben.

Ein Überschuss an der Ketenverbindung (III.1) kann zu unerwünschten Nebenreaktionen führen. Daher erfolgt die Umsetzung der Verbindung der allgemeinen Formel (II.1) mit dem Keten (III.1) vorzugsweise unter Verwendung von höchstens äquimolaren Mengen an der Ketenverbindung (III.1). Bevorzugt ist ein geringer molarer Überschuss an der Verbindung der allgemeinen Formel (II.1).

Vorzugsweise erfolgt die Umsetzung der 4-Hydroxy-tetrahydropyranverbindung der allgemeinen Formel (II.1) mit dem Keten (III.1) so, dass eine Akkumulation der Ketenverbindung im Reaktionsgemisch zu jedem Zeitpunkt der Umsetzung vermieden wird.

Bevorzugt erfolgt die Umsetzung der Verbindung der allgemeinen Formel (II.1) mit dem Keten (III.1) so, dass so lange Keten in das Reaktionsgemisch eingeleitet wird, bis die Verbindung (II.1) im Wesentlichen vollständig umgesetzt ist. Unter "im Wesentlichen umgesetzt" wird dabei ein Umsatz von wenigstes 98 %, bevorzugt von wenigstens 99 %, verstanden.

Bevorzugt wird die Verbindung der allgemeinen Formel (II.1) einer Umsetzung mit Keten (III.1) bei einer Temperatur im Bereich von 0 bis 150 °C, bevorzugt von 10 bis 120 °C, unterzogen.

In einer ersten bevorzugten Ausführungsform wird die Verbindung der allgemeinen Formel (II.1) einer Umsetzung mit Keten (III.1) in Abwesenheit eines zugesetzten Katalysators unterzogen.

In einer zweiten bevorzugten Ausführungsform wird die Verbindung der allgemeinen Formel (II.1) einer Umsetzung mit Keten (III.1) in Gegenwart eines Katalysators unterzogen. Bevorzugt wird wenigstens ein Zinksalz als Katalysator eingesetzt, das auch als Hydrat bzw. Mehrfach-Hydrat vorliegen kann.

Besonders bevorzugt wird als Katalysator ein Zinksalz einer Carbonsäure, speziell einer Monocarbonsäure mit 1 bis 18 C-Atomen oder Dicarbonsäure mit 2 bis 18 C-Atomen, eingesetzt. Dazu zählen z. B. Zinkformiat, Zinkacetat, Zinkpropionat, Zinkbutyrat, Zinkstearat, Zinksuccinat oder Zinkoxalat. Besonders bevorzugt ist Zinkacetat.

Sehr vorteilhaft am Verfahren ist, dass man die Katalysatoren in der Regel nur in sehr kleinen Mengen einsetzen muss, was das Verfahren kostengünstiger macht und die Aufarbeitung des Reaktionsgemisches erleichtert. Dies gilt insbesondere für den Einsatz eines Zinksalzes als Katalysator.

Bevorzugt verwendet man den Katalysator in einer Menge von 0,01 bis 2 Gew.-%, besonders bevorzugt von 0,02 bis 0,5 Gew.-%, bezogen auf die Gesamtmenge der Verbindung (II.1).

Zur Durchführung der Umsetzung geht man mit Vorteil so vor, dass man diese Umsetzung in einem geeigneten Reaktionsgefäß durchführt, welches als wesentliche Bestandteile eine gute Rühr- und/oder Mischeinrichtung, eine Dosiervorrichtung für Keten, eine Heizvorrichtung zum Starten der Reaktion und zum Aufrechterhalten der Reaktionstemperatur während der Nachreaktion, eine Kühleinrichtung zum Abführen der Reaktionswärme der exothermen Umsetzung sowie eine Vakuumpumpe enthält.

Für eine optimale Reaktionsführung ist es vorteilhaft, dass man das Keten so zudosiert, dass es im Reaktionsgemisch nie im Überschuss vorliegt, und dass das Reaktionsgemisch immer gut durchmischt wird.

Für eine optimale Reaktionsführung ist es weiterhin vorteilhaft, dass man zu schnelles Zudosieren von Keten vermeidet sowie das Ende der Umsetzung eindeutig feststellt.

Der Nachweis von Keten gelingt beispielsweise IR-spektroskopisch über die charakteristische Carbonylschwingung.

Mit Hilfe des beschriebenen Verfahrens ist es möglich, die Verbindungen der allgemeinen Formel (1.1) auf technisch einfache Weise in hohen Reinheiten und dennoch in ausgezeichneten Ausbeuten und Raum-Zeit-Ausbeuten herzustellen. Da die Edukte im Wesentlichen vollständig zu Produkten umgesetzt werden, zeichnet sich das beschriebene Verfahren durch eine maximale Atomökonomie aus.

Die nach dem beschriebenen Verfahren erhältlichen Zusammensetzungen eignen sich besonders vorteilhaft als Riechstoff oder zur Bereitstellung eines Riechstoffs.

Die Zusammensetzungen können für den Einsatz als Riechstoff mit wenigstens einem auf diesem Anwendungsgebiet üblichen Lösemittel beliebig verdünnt werden. Beispielhaft seien als geeignete Lösemittel genannt: Ethanol, Dipropylenglycol oder dessen Ether, Phthalate, Propylenglykole oder Carbonate von Diolen, bevorzugt Ethanol. Auch Wasser ist als Lösemittel zur Verdünnung der erfindungsgemäßen Duftstoffkompositionen geeignet und kann vorteilhaft zusammen mit geeigneten Emulgatoren eingesetzt werden.

Die nach dem beschriebenen Verfahren erhaltenen Riechstoffe weisen auf Grund der strukturellen und chemischen Ähnlichkeit der Komponenten eine hohe Stabilität und Haltbarkeit auf.

Die nach dem beschriebenen Verfahren erhaltenen Riechstoffe eigenen sich zur Einarbeitung in kosmetische Zusammensetzungen sowie Gebrauchs- und Verbrauchsgüter bzw. Mittel, wie sie im Folgenden näher beschrieben sind, wobei die Riechstoffe in die genannten Güter eingearbeitet oder auch auf solche aufgebracht sein können. Unter einer organoleptisch wirksamen Menge ist dabei, wie im Rahmen der gesamten vorliegenden Erfindung, insbesondere eine solche Menge zu verstehen, die bei sachgemäßer Anwendung ausreicht, beim Anwender bzw. Verbraucher einen Dufteindruck hervorzurufen.

Als kosmetische Zusammensetzungen sind alle üblichen kosmetischen Zusammensetzungen geeignet. Dabei handelt es sich bevorzugt um Parfum, Eau de Toilette, Deodorants, Seife, Duschgel, Badegel, Crèmes, Lotions, Sonnenschutzmittel, Zusammensetzungen zur Reinigung und Pflege der Haare, wie Haarshampoo, Spülung, Haargel, Haarfestiger in Form von Flüssigkeiten oder Schäumen und weitere Reinigungs- oder Pflegemittel für die Haare, Zusammensetzungen zur dekorativen Anwendung am menschlichen Körper, wie kosmetische Stifte, zum Beispiel Lippenstifte, Lippenpflegestifte, Abdeckstifte (Concealer), Wangenrouge (Blusher), Lidschattenstifte, Lippenkonturenstifte, Augenkonturenstifte, Augenbrauenstifte, Korrekturstifte, Sonnenschutzstifte, Anti-Akne-Stifte und vergleichbare Produkte sowie Nagellacke und weitere Produkte zur Nagelpflege.

Die nach dem beschriebenen Verfahren erhaltenen Riechstoffe eignen sich speziell für einen Einsatz in Parfums, z. B. als Eau de Toilette, Duschgele, Badegele und Körperdeodorants.

Sie eignen sich weiterhin zur Aromatisierung von Verbrauchs- oder Gebrauchsgütern, in die sie eingearbeitet bzw. auf die sie aufgebracht werden und ihnen dadurch einen angenehmen frischen grünen Akzent verleihen. Beispiele für Verbrauchs- oder Gebrauchsgüter sind: Raumluftdeodorants (Air Care), Reinigungsmittel oder Pflegemittel für Textilien (speziell Waschmittel, Weichspüler), Textilbehandlungsmittel wie beispielsweise Bügelhilfsmittel, Putzmittel, Reinigungsmittel, Pflegemittel zur Behandlung von Oberflächen, beispielsweise von Möbeln, Fußböden, Kücheneinrichtungen, Glasscheiben und Fenstern sowie Bildschirmen, Bleichen, Toilettensteine, Entkalkungsmittel, Düngemittel, Baustoffe, Schimmelentferner, Desinfektionsmittel, Produkte für die Auto- bzw. Fahrzeugpflege und dergleichen mehr.

### BEISPIELE

Gaschromatographische Analysen wurden nach der folgenden Methode durchgeführt:
Säule: DB WAX 30 m x 0,32 mm
FD 0,25 µm
Injektortemperatur: 200 °C; Detektortemperatur 250 °C
Temperaturprogramm: Anfangstemp.: 60 °C, mit 2 °C/min auf 120 °C,
   mit 20 °C/min auf 230 °C
Retentionszeiten:
   trans-Tetrahydro-2-isobutyl-4-methylpyranyl-4-acetat (nicht erfindungsgemäß) t_{R} = 15,1 min
   cis-Tetrahydro-2-isobutyl-4-methylpyranyl-4-acetat (nicht erfindungsgemäß) t_{R} = 18,8 min
   trans-Tetrahydro-2-isobutyl-4-methylpyran-4-ol (nicht erfindungsgemäß) t_{R} = 19,6 min cis-Tetrahydro-2-isobutyl-4-methylpyran-4-ol (nicht erfindungsgemäß) t_{R} = 21,5 min

Die Konzentrationen der erhaltenen Produkte (Gew.-%) wurden GC-analytisch mit internem Standard ermittelt.

### Beispiel 1 (nicht erfindungsgemäß):

(Herstellung von Tetrahydro-2-isobutyl-4-methylpyranyl-4-acetat aus Tetrahydro-2-isobutyl-4-methylpyran-4-ol durch Umsetzung mit Keten)

127,08 g Tetrahydro-2-isobutyl-4-methylpyran-4-ol (0,74 Mol; Zusammensetzung vgl. Tabelle 1, Probe nach 0 h) wurden bei 90 °C vorgelegt. Keten, das durch die Pyrolyse von Aceton (0,411 mL/min) bei 700 °C erhalten wurde, wurde nach Abkühlen bei 90 °C unterhalb der Flüssigkeitsoberfläche und unter kräftigem Rühren eingeleitet. Der Umsatz der Pyrolyse lag bei ca. 48 % (bezogen auf isoliertes, nicht umgesetztes Aceton) und der Ketengehalt im Pyrolysegas lag bei 23 bis 24 %. Nach 7 h Reaktionszeit wurde die Keten-Einleitung unterbrochen und am Folgetag fortgesetzt. Nach insgesamt 10 h Reaktionszeit war das Startmaterial umgesetzt, und der Versuch wurde beendet. Insgesamt wurden während des Versuchs 92,6 g Aceton (1,59 Mol, 2,15 Äq.; bezogen auf isoliertes, nicht umgesetztes Aceton) in der Pyrolyse umgesetzt. Die Zusammensetzung der Proben ist in Tabelle 1 angegeben. Die Ausbeute an Tetrahydro-2-isobutyl-4-methyl-pyranyl-4-acetat lag bei 89 %.

Cis- und trans-Tetrahydro-2-isobutyl-4-methylpyranyl-4-acetatwurden per NMR-Spektroskopie charakterisiert:
Cis-Tetrahydro-2-isobutyl-4-methylpyranyl-4-acetat: ¹³C NMR (125 MHz, CDCl₃): δ = 21.7, 22.3, 22.5, 23.2, 24.3, 37.7, 43.8, 45.4, 64.6, 72.7, 80.0, 170.3 ppm.
Trans-Tetrahydro-2-isobutyl-4-methylpyranyl-4-acetat: ¹³C NMR (125 MHz, CDCl_{3):} δ = 22.37, 22.39, 23.2, 24.3, 26.2, 36.3, 42.5, 45.1, 63.4, 70.9, 79.3, 170.4 ppm.

**Tabelle 1: Zusammensetzung des Reaktionsgemisches**

| Reaktionszeit | trans-Tetrahydro-2-isobutyl-4-methylpyranyl-4-acetat | cis-Tetrahydro-2-isobutyl-4-methylpyranyl-4-acetat | Summe Tetrahydro-2-isobutyl-4-methylpyranyl-4-acetat | trans-Tetrahydro-2-isobutyl-4-methylpyran-4-ol | cis-Tetrahydro-2-isobutyl-4-methylpyran-4-ol | Summe Tetrahydro-2-isobutyl-4-methylpyran-4-ol |
|---|---|---|---|---|---|---|
| [h] | [%]* | [%]* | [%]* | [%]* | [%]* | [%]* |
| 0 | 0 | 0 | 0 | 22,8 | 77,1 | 99,9 |
| 2 | 3,1 | 16,0 | 19,1 | 16,9 | 52,9 | 69,8 |
| 4 | 7,4 | 35,0 | 42,4 | 15,6 | 34,5 | 50,1 |
| 6 | 11,6 | 49,8 | 61,4 | 13,7 | 18,9 | 32,6 |
| 7 | 15,2 | 60,7 | 75,9 | 10,6 | 8,4 | 19,0 |
| 8 | 18,6 | 73,4 | 92,0 | 1,5 | 2,4 | 3,9 |
| 9 | 20,6 | 74,4 | 95,0 | 0 | 0,4 | 0,4 |
| 10 | 21,2 | 73,9 | 95,1 | 0 | 0 | 0 |

| | | | | | | |
|---|---|---|---|---|---|---|
| * GC-Gew.-% | | | | | | |

## Patentansprüche

1. Verbindungen der allgemeinen Formel (1.1) 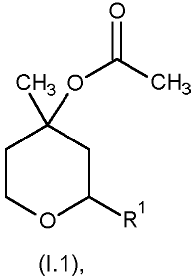 worin
R¹ für Phenyl oder mit C₁-C₆-Alkyl oder C₁-C₆-Alkoxy substituiertes Phenyl steht.

2. Verbindung gemäß Anspruch 1, ausgewählt unter Verbindungen der Formeln (I-A.1), (I-A.6), (I-A.7), (I-A.8) und (I-A.9)

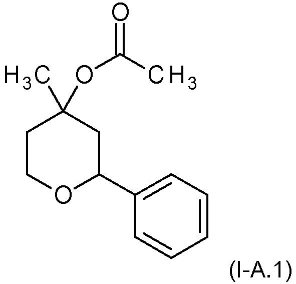

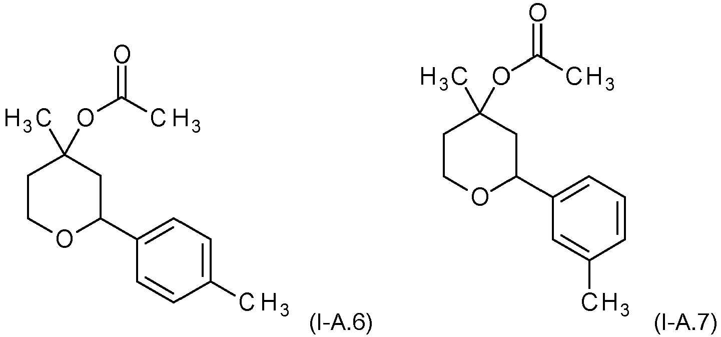

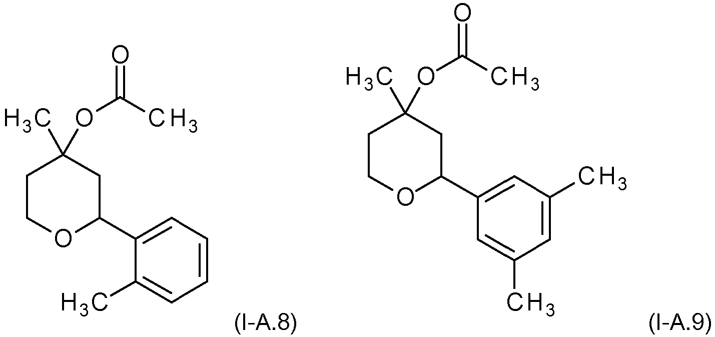

3. Verwendung von Verbindungen der Formel (1.1) gemäß einem der Ansprüche 1 oder 2 als Riechstoff.

## Claims

1. A compound of the general formula (I.1) 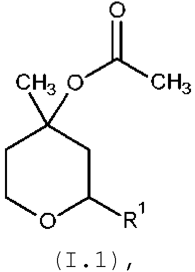 in which
R¹ is phenyl or C₁-C₆-alkyl- or C₁-C₆-alkoxy-substituted phenyl.

2. The compound according to claim 1, selected from compounds of formulae (I-A.1), (I-A.6), (I-A.7), (I-A.8) and (I-A.9)

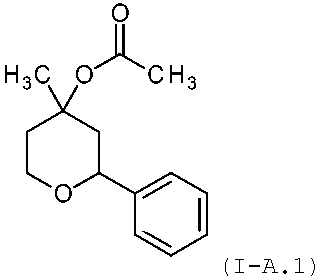

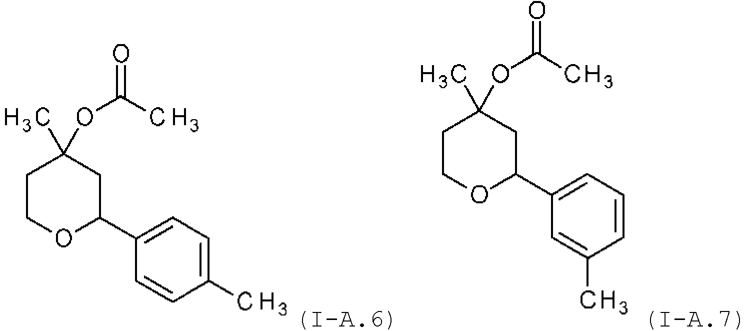

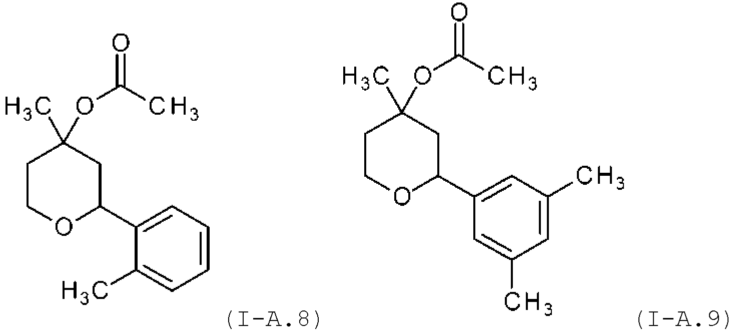

3. The use of compounds of formula (I.1) according to either of claims 1 and 2 as an odorant.

## Revendications

1. Composés de formule générale (I.1) 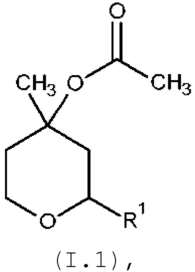 dans laquelle
R¹ représente phényle ou phényle substitué par C₁-C₆-alkyle ou C₁-C₆-alcoxy.

2. Composé selon la revendication 1, choisi parmi les composés des formules (I-A.1), (I-A.6), (I-A.7), (I-A.8) et (I-A.9)

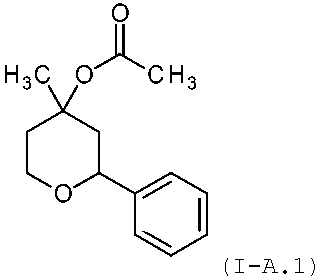

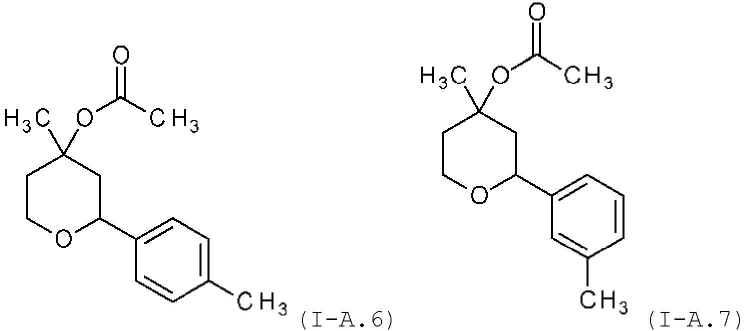

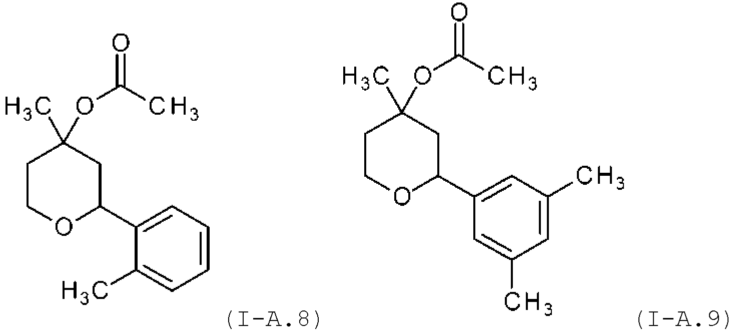

3. Utilisation de composés de formule (I.1) selon l'une des revendications 1 à 2 comme substance odorante.
